# EUROPEAN PATENT SPECIFICATION

(11) **EP 3 015 089 B1**
(45) Date of publication and mention of the grant of the patent: **14.10.2020**
(21) Application number: 15194577.1
(22) Date of filing: 02.12.2008
(51) Int. Cl.: A61F 2/00, D04B 21/12

(54) **IMPLANT FOR PARASTOMAL HERNIA**
IMPLANTAT FÜR PARASTOMALE HERNIEN
IMPLANT POUR HERNIE PARASTOMIALE

(30) Priority: 03.12.2007 FR 0708429; 03.12.2007 US 5131
(43) Date of publication of application: 04.05.2016
(62) Divisional of application: 08855777.2
(73) Proprietor: Sofradim Production, 01600 Trevoux (FR)
(72) Inventor: LECUIVRE, Julie, 69620 LE BOIS D'OINGT (FR); MENEGHIN, Alfredo, 69006 Lyon (FR); THERIN, Michel, 69004 LYON (FR); SPINNLER, Linda, 69480 POMMIERS (FR)
(74) Representative: Delorme, Nicolas

(56) References cited:
- EP-A1- 0 621 014
- WO-A1-01/80773
- WO-A1-02/22047
- US-A1- 2003 212 460
- US-A1- 2007 250 147

## Description

The present invention relates to an implant suitable for use in the prevention and/or treatment of hernias that may occur in the area of a stoma, particularly one formed in the abdominal wall.

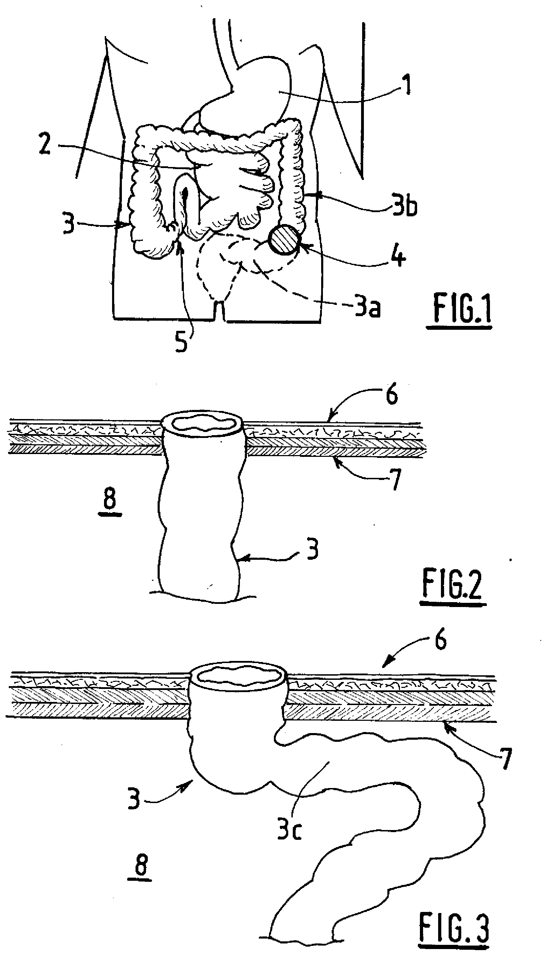

Stomas are openings formed in a wall, for example the abdominal wall, for joining a hollow organ, for example the intestine, to the skin. Such an operation proves necessary, for example in cases of cancer of the rectum or Crohn's disease, to create an artificial anus for example, during which operation the diseased part of the intestine is resected and the healthy intestine is exteriorized at the skin. In this case, the stoma is formed in the abdominal wall. Figure 1 is a schematic illustration of the human digestive tract. This diagram shows the stomach 1, the small intestine 2 and the colon 3. The broken lines represent the part 3a of the colon that is diseased and has been removed during the surgical procedure. The healthy part 3b of the colon now opens to the outside at the stoma 4 formed in the abdominal wall. Depending on the extent of the diseased part of the colon, the stomas can be formed in the area of the ileum 5 (ileostomy) or of the colon (colostomy), as shown in Figure 1.

Stomas can also be formed in the area of the ureters (ureterostomy).

After operations of this kind, hernias may develop around the stoma, that is to say in the area of the peristomal wall. A weakening of the wall around the stoma may therefore result in the appearance of a parastomal hernia. To treat these parastomal hernias, prostheses are implanted that are designed to strengthen the abdominal wall inside the patient, in the area of the stoma. The implantation of these prostheses can be intraperitoneal, that is to say within the actual abdominal wall, or retroperitoneal, resting against the abdominal wall.

Prostheses for treating parastomal hernias have been described in the document WO2004/071349. However, these prostheses are not entirely satisfactory, particularly since they are not adapted to all types of stomas that are formed, particularly indirect stomas.

The reason is that, for example in the case of the colon, several stoma configurations can be formed: the direct stoma, as shown in Figure 2, in which the colon 3 issuing from the abdominal cavity 8 is perpendicular to the abdominal wall 7 and hence to the skin 6 prior to its exteriorization, or the indirect stoma, as shown in Figure 3, in which the colon 3 is caused to form a bend within the abdominal cavity 8 prior to its exteriorization, the colon thus having a part 3c parallel to the abdominal wall 7. The indirect stoma avoids a situation where the internal part of the colon in the area of the stoma becomes invaginated and exteriorizes.

WO0180773 discloses a textile having first and second surfaces wherein the textile has a first part having a second-dimensional knit and a second part having a third-dimensional knit. It is disclosed as being particular suitable for treating hernias.

US2003212460 discloses an hernia implant having an anti-adhesive film or barrier on the surface facing the abdominal cavity when placed in the body.

There is therefore a need for a parastomal prosthesis able to protect the intestine and hollow organs and to effectively strengthen the abdominal wall regardless of the type of stoma that has been formed.

The present invention aims to meet this need by making available an implant that has specific surfaces able to protect the hollow organs, such as the intestine, regardless of the stoma that has been formed, and at the same time to effectively strengthen the abdominal wall. The invention is defined as in claim 1; further advantageous embodiments are defined in the dependent claims.

The subject matter of the present invention is an implant for the prevention or treatment of a hernia formed in the abdominal wall in the proximity of a stoma of an organ, comprising a layer of a porous structure whose surface intended to face the abdominal cavity is covered by a first film of anti-adhesive material, characterized in that said porous structure comprises a first part intended to be in contact with the stoma organ and having a first thickness E1, and a second part having a second thickness E2 greater than said first thickness E1, said first part being covered, on its surface intended to face the abdominal wall, by a second film of anti-adhesive material.

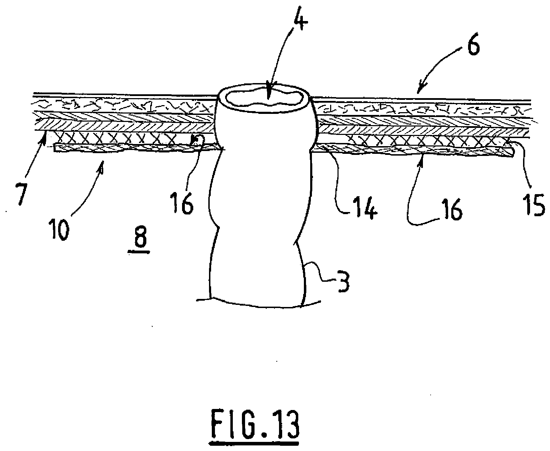

Thus, in the implant according to the invention, the first part of the porous structure, the part intended to be in contact with the stoma organ, for example in contact with the intestine, is covered by a film of anti-adhesive material on both of its surfaces. In one embodiment of the invention, the first and second films of anti-adhesive material are joined to form just one film, and the first part of porous structure is totally enclosed within the film of anti-adhesive material. As will become clear from the explanations given later with reference to Figures 13 to 15, the stoma organ, for example the intestine, is protected irrespective of whether the stoma is a direct or indirect one, because the part of the implant able to come into contact with it is covered by a film of anti-adhesive material.

In the present application, an "implant" is understood as a biocompatible medical device that can be implanted in the human or animal body.

Within the meaning of the present application, the word "porous" is understood as the characteristic according to which a structure has pores or meshes, pockets, holes or orifices, that are open and are distributed uniformly or irregularly and promote all cell colonization. The pores can be present in all types of configurations, for example as spheres, channels, hexagonal forms.

According to one embodiment of the invention, the porous structure comprises a sponge, a fibrous matrix or a combination of a sponge and of a fibrous matrix. For example, the sponge can be obtained by lyophilization of a gel, with pores being created during the lyophilization. The fibrous matrix can be any arrangement of yarns or yarn portions creating pores between the yarns and/or yarn portions. The fibrous matrix of the present invention is a textile obtained by knitting.

In one embodiment of the invention, the porous structure, for example the sponge and/or the fibrous matrix, has pores with dimensions ranging from approximately 0.1 to approximately 3 mm.

According to one embodiment of the invention, said thickness E1 of the first part of the porous structure ranges from approximately 0.15 to 0.50 mm. A relatively small thickness of this kind allows the abdominal wall to be strengthened without any risk of damaging the stoma organ, for example the intestine, which is in contact with the implant.

Said first part of porous structure is a textile in the form of a knit. This knit is a two-dimensional knit, that is to say preferably a knit having a thickness less than or equal to 5 times the mean diameter of the yarns from which it is made, for example knitted on a warp knitting machine or raschel machine with the aid of two guide bars forming a knit with two surfaces, said knit being free of sheets of connecting yarns between its two opposite surfaces.

The pores of the two-dimensional knit are formed by the empty spaces situated between the constituent yarns of the knit, for example the meshes.

The constituent yarns of the knit that form the first part of porous structure can be chosen from among yarns made of biocompatible materials, bioabsorbable materials, non-bioabsorbable materials and their mixtures.

In the present application, the word "bioabsorbable" is understood as the characteristic according to which a material is absorbed by the biological tissues and disappears *in vivo* at the end of a given period, which can vary for example from one day to several months, depending on the chemical nature of the material.

Thus, examples of bioabsorbable materials suitable for the yarns forming the first part of porous structure are polylactic acid (PLA), polysaccharides, polycaprolactones (PCL), polydioxanones (PDO), trimethylene carbonates (TMC), polyvinyl alcohol (PVA), polyhydroxyalkanoates (PHA), polyamides, polyethers, oxidized cellulose, polyglycolic acid (PGA), copolymers of these materials and their mixtures.

Examples of non-bioabsorbable materials suitable for the yarns forming the first part of porous structure are polypropylenes, polyesters such as polyethylene terephthalates, polyamides, polyvinylidene fluoride, and their mixtures.

The yarns forming the first part of porous structure of the implant can, for example, be chosen from among monofilament yarns, multifilament yarns and their combinations.

The multifilament yarn count preferably varies from 40 to 110 dtex.

The monofilament yarns preferably have a diameter ranging from 0.06 to 0.15 mm.

In one embodiment of the invention, the yarns forming the first part of porous structure are monofilament yarns. Such monofilament yarns pose less risk of sepsis than do multifilament yarns.

In one embodiment of the invention, the monofilament yarns are of polyethylene terephthalate.

A monofilament yarn suitable for the first textile part of the implant according to the invention is, for example, a monofilament yarn with a diameter of approximately 0.08 mm, of polyethylene terephthalate.

The porous structure of the implant according to the invention comprises a second part with a thickness E2 greater than the thickness E1 of the first part. The second part of porous structure is essentially designed to act as a reinforcement of the abdominal wall.

Thus, the value of the thickness E2 of the second part of porous structure can vary depending on the value of the thickness E1 of the first part of the structure, the value of the thickness E2 of the second part of porous structure needing to be greater than that of the value of the thickness E1 of the first part of porous structure. The reason is that the second part of porous structure preferably has mechanical strength superior to that of the first part of porous structure. For example, said second thickness E2 of the second part of the porous structure can range from approximately 0.40 to 3.00 mm.

As will become clear from the description that follows, the surface of the layer of porous structure intended to be placed facing the abdominal cavity is covered by a film of anti-adhesive material which prevents the organs and other viscera of the abdominal cavity from attaching themselves to the implant. This surface will be referred to hereinafter as the closed surface of the implant. By contrast, the surface of the second part of porous structure intended to be placed facing the abdominal wall is not covered by a film of anti-adhesive material and remains open to all cell colonization at the time of implantation. This surface will be referred to hereinafter as the open surface of the second part of porous structure. This surface of the second part of porous structure is intended to be placed resting against the abdominal wall. To permit better fixing of the implant to the abdominal wall, the open surface of the second part of porous structure can comprise fastening means, for example self-fixing ones, inherent to this surface.

Thus, by virtue of its porous character and its thickness, the second part of porous structure of the implant according to the invention is especially adapted to promote tissue growth via its open surface after implantation. The cells of the abdominal wall deeply colonize the second part of porous structure by way of its open surface placed facing the abdominal wall.

In the invention, the second part of porous structure is a textile in the form of a three-dimensional knit, for example as described in applications WO99/06080 and WO99/05990. Within the meaning of the present application, the term "three-dimensional knit" is understood as an assembly or arrangement of monofilament or multifilament yarns or a combination of these, obtained by knitting and having two opposite surfaces that are separated by a significant thickness, preferably of greater than or equal to 0.50 mm, said thickness comprising connecting yarns and pores.

Such a three-dimensional knit can be knitted, for example, on a warp knitting machine or double-bed raschel machine with the aid of several guide bars forming a knit that comprises two opposite surfaces and a spacer. In the present application, the word "spacer" is understood as the set or sets of yarns that connect the two surfaces of a three-dimensional knit to each other, thereby constituting the thickness of a knit, as is described in WO99/06080 or in WO99/05990.

Thus, in the case where the second part of porous structure is a three-dimensional knit as described above, the knitting structure can define within the thickness of the knit a multiplicity of transverse channels or pockets that may or may not be mutually parallel. These pockets or channels can be interconnected and thus allow the colonizing cells to pass from one pocket or channel to another. A second part of porous structure of this type promotes good tissue growth after implantation.

The yarns constituting the second part of porous structure of the implant according to the invention can be chosen from among yarns made of biocompatible materials, bioabsorbable materials, non-bioabsorbable materials and their mixtures, already listed above for the first part of porous structure.

Thus, examples of bioabsorbable materials suitable for the yarns forming the second part of porous structure are polylactic acid (PLA), polysaccharides, polycaprolactones (PCL), polydioxanones (PDO), trimethylene carbonates (TMC), polyvinyl alcohol (PVA), polyhydroxyalkanoates (PHA), polyamides, polyethers, oxidized cellulose, polyglycolic acid (PGA), copolymers of these materials and their mixtures.

Examples of non-bioabsorbable materials suitable for the yarns forming the second part of porous structure are polypropylenes, polyesters such as polyethylene terephthalates, polyamides, polyvinylidene fluoride, and their mixtures.

The yarns forming the second part of the porous structure can, for example, be chosen from among monofilament yarns, multifilament yarns and their combinations.

The multifilament yarn count preferably varies from 40 to 110 dtex.

The monofilament yarns preferably have a diameter ranging from 0.06 to 0.15 mm.

In one embodiment of the invention, the yarns forming the first part of porous structure are monofilament yarns. Such monofilament yarns pose less risk of sepsis than do multifilament yarns. For example, the monofilament yarns are of polyethylene terephthalate.

A monofilament yarn suitable for the second part of porous structure of the implant according to the invention is, for example, a monofilament yarn with a diameter of approximately 0.08 mm, of polyethylene terephthalate.

In one embodiment of the invention, said second part of porous structure has, on its open surface intended to face the abdominal wall, means of fastening said second part to said abdominal wall. These fastening means can be chosen from among elements that are integrally formed on said second textile part, such as loops and barbs, or from among elements joined to the surface of the second textile part, such as a rough covering, hooks, threads or clips fixed on the surface of the second textile part.

In one embodiment of the invention, said fastening means are chosen from among loops, barbs and their mixtures. In such a case, the loops and barbs can be obtained from yarns or portions of yarns that are woven and/or knitted directly for example, with the three-dimensional knit forming the second part of porous structure. For example, in order to obtain barbs, it is possible to use hot-melt yarns such as are described in the application WO01/81667.

In the invention, in which the first part of porous structure is in the form of a two-dimensional knit and the second part of porous structure is in the from of a three-dimensional knit, the two knits, i.e. two-dimensional and three-dimensional, can be manufactured separately then joined together by at least one seam, for example, in order to form the layer of porous structure of the implant.

In another embodiment, the two-dimensional knit and the three-dimensional knit are knitted together on the same knitting machine and constitute a textile made in one piece, for example by using supplementary guide bars for the three-dimensional knit and/or different yarn runs for producing each of the two knits. In such an embodiment of the invention, the porous structure layer of the implant according to the invention is composed of a textile formed in one piece, said textile having a two-dimensional zone, corresponding to the first part of the porous structure, and one or more three-dimensional zones, corresponding to the second part of the porous structure. In such an embodiment, it is possible to form a selvage at the passage from a two-dimensional zone to a three-dimensional zone with a view to forming a smooth connection between the two parts, such that the difference in thickness between the two parts does not form a step that could damage the biological tissue situated in the proximity of the implant.

The layer of porous structure of the implant according to the invention is covered, on its second surface intended to face the abdominal cavity, by a first film of anti-adhesive material. Moreover, the first part of porous structure is covered, on its surface intended to face the abdominal wall, by a second film of anti-adhesive material.

Within the meaning of the present application, the term "anti-adhesive material" is understood as a smooth and non-porous biocompatible material that prevents the organs and other viscera of the abdominal cavity from attaching themselves to the implant.

The anti-adhesive material forming the first film can be identical to or different from the material forming the second film.

In one embodiment of the invention, the anti-adhesive material constituting the first and/or second film(s) is chosen from among bioabsorbable materials, non-bioabsorbable materials and their mixtures.

In one embodiment of the invention, the bioabsorbable materials suitable for the first and/or second film(s) of anti-adhesive material are chosen from among collagens, oxidized celluloses, polyarylates, trimethylene carbonates, caprolactones, dioxanones, glycolic acid, lactic acid, glycolides, lactides, polysaccharides, for example chitosans, polyglucuronic acids, hylauronic acids, dextrans and their mixtures.

In one embodiment of the invention, the non- bioabsorbable materials suitable for the first and/or second film of anti-adhesive material are chosen from among polytetrafluoroethylene, polyethylene glycols, polysiloxanes, polyurethanes, stainless steels, derivatives of precious metals and their mixtures.

In one embodiment of the invention, the material constituting the first and/or second film(s) of anti-adhesive material is a hydrophilic bioabsorbable material, preferably chosen from the group formed by collagens, polysaccharides and their mixtures. Of the collagens that can be used according to the invention, the following may be mentioned:
1) collagen whose helical structure is at least partially denatured by heat, without hydrolytic degradation, and whose method of preparation is described in WO99/06080,
2) native collagen, not heated, filmed with or without glycerol, crosslinked by gamma irradiation or by other chemical or physical means,
3) and/or their mixtures.

Of the polysaccharides that can be used as absorbable hydrophilic material according to the invention, the following may be mentioned: oxidized cellulose, hylauronic acid, starch, chitosan, crosslinked dextrans and/or their mixtures. All these materials are well known to persons skilled in the art. An oxidized cellulose suitable for the present invention is the product sold under the brand name "Interceed®" by Ethicon. A hyaluronic acid suitable for the present invention is the product sold under the brand name "Hyalobarrier®" by Fidia Advanced Biopolymers, or the product sold under the brand name "Seprafilm®" by Genzyme.

In one embodiment of the invention, the first film and the second film form a single and unique film, the first film then completely coating the first part of porous structure and thus covering this porous structure part both on its surface intended to face the abdominal cavity and also on its surface intended to face the abdominal wall. Thus, said first part of porous structure is totally enclosed in the film of anti-adhesive material before implantation and at the moment of implantation.

Thus, at the moment of implantation, and whatever the embodiment of the invention, the two surfaces of the first part of porous structure are occluded by a continuous film of anti-adhesive material.

The first part of the porous structure of the implant according to the invention, regardless of whether it is totally coated by the first film of anti-adhesive material or whether each of its surfaces are covered, one by the first film of anti-adhesive material, the other by the second film of anti-adhesive material, is thus protected at least during the initial phase of cicatrization, i.e. is not exposed to the inflammatory cells such as granulocytes, monocytes, macrophages, or the multinucleated giant cells that are generally activated by the surgical procedure. Nor is it exposed to the bacteria that may be present. The reason for this is that, at least during the initial phase of cicatrization, which may last approximately 5 to 10 days, only the film or films of anti-adhesive material are accessible to the various factors such as proteins, enzymes, cytokines or inflammatory cells, in the first textile part.

In the case where the film or films of anti-adhesive material are made of non-absorbable materials, they thus protect the first part of porous structure before and after implantation, throughout the period of implantation of the implant.

Furthermore, by virtue of the film or films of anti-adhesive material, the surrounding fragile tissues, such as the hollow viscera for example, are protected in particular from the formation of severe postsurgical fibrous adhesions.

In the case where the anti-adhesive material comprises a bioabsorbable material, it is preferable to choose a bioabsorbable material that is not absorbed until after a few days, such that the film of anti-adhesive material can perform its function of protecting the stoma organ, for example the intestine, and the hollow organs during the days following the operation, and until the cellular recolonization of the implant in turn protects the fragile organs.

The thickness of the first anti-adhesive film is preferably much less than the thickness E2 of the second part of porous structure. In fact, the film of anti-adhesive material must preferably not occlude the open surface of the second part of the porous structure, so as to permit cellular recolonization of the second part of porous structure after implantation.

The first film of anti-adhesive material is preferably continuous, smooth and non-porous, covering the whole surface of the porous structure intended to be placed facing the abdominal cavity. In one embodiment, the first film of anti-adhesive material extends past the edges of the layer of porous structure. Thus, the implant is protected from contact with the viscera. The first film of anti-adhesive material can, for example, extend past the edges of the layer of porous structure by a distance ranging from 3 to 10 millimetres.

The first film of anti-adhesive material is preferably joined to the surface of the layer of porous structure intended to be placed facing the abdominal cavity by means of surface penetration, keeping open the porosity on the opposite surface of the second part of the porous structure, that is to say the open surface, intended to be placed facing the abdominal wall.

The implant according to the invention can be used via the laparoscopic route. If necessary, for example when the first and second films of anti-adhesive material are made of dried collagen, it is preferable to rehydrate the implant at the time of use, in order to make it flexible and easier to use.

The implant according to the invention can, for example, be prepared according to the following method:
a) a textile is prepared that has two-dimensional zones and three-dimensional zones, as has been described above,
b) a solution of an anti-adhesive material is prepared,
c) the solution obtained at b) is poured into a mould,
d) the textile is then applied to the solution, the surface of the textile intended to face the abdominal cavity being placed on said solution in such a way that said solution impregnates the two-dimensional zones of said textile completely,
e) it is left to dry.

With such a method it is possible to obtain an implant according to the invention in which the first film and the second film form a single and unique film.

Alternatively, step d) is replaced by step d') in which the solution of anti-adhesive material only superficially impregnates a single surface of the two-dimensional zones, thereby forming the first film. The procedure is then supplemented by an additional step in which the opposite surface of the two-dimensional zones is impregnated by the same solution of anti-adhesive material or by another solution of another anti-adhesive material in order to form the second film.

Methods of covering/coating that can be used according to the present invention are described in documents WO99/06080 and WO2004/043294.

The implant according to the invention can have any shape adapted to the anatomy of the patient and/or to the surgical technique envisaged. For example, the shape of the implant can be round, oval, square or rectangular.

In one embodiment, the implant has a generally elongate shape, for example oval or rectangular. For example, the length of the implant can range from 12 to 30 cm and its width can range from 10 to 20 cm.

In another embodiment, the implant has a generally round shape. For example, the diameter of the implant can range from 5 to 20 cm.

In one embodiment of the invention, said first part of porous structure has the form of a central strip, and, for example, the width of the central strip can range from 2 to 10 cm.

In another embodiment of the invention, said first part of porous structure has the form of a disc, and, for example, the diameter of the disc can range from 2 to 10 cm.

In one embodiment of the invention, at least one orifice is formed at the centre of the first part of the porous structure in order to provide a passage for the stoma organ, for example the intestine, during implantation of the implant. Alternatively, at least one orifice is formed within the first part of the porous structure, said orifice being offset relative to the centre of the implant. For certain types of surgery, for example ureterostomies, the implant can have two orifices. In one embodiment of the invention, the orifice or orifices can be connected to an edge of the implant by way of a slit. For example, the dimensions of the orifices can range from 0.5 to 8 cm. The orifice or orifices can be offset relative to the centre of the implant.

The advantages of the present invention, and variants thereof, will become evident from the following detailed description and from the attached drawings, in which:
Figure 1 is a schematic illustration of the human digestive tract, in which a stoma has been formed,
Figure 2 is a schematic illustration of a direct stoma,
Figure 3 is a schematic illustration of an indirect stoma,
Figure 4 is a plan view of a first embodiment of an implant according to the invention,
Figure 5 is a plan view of a second embodiment of an implant according to the invention,
Figure 6 is a simplified schematic cross-sectional view of the implant from Figure 4,
Figure 7 is a photograph taken with a Hitachi S-800 FEG scanning electron microscope, magnification x40, showing an embodiment of the first part of porous structure of an implant according to the invention,
Figure 8 is a photograph taken with a Hitachi S-800 FEG scanning electron microscope, magnification x250, showing the first part of porous structure from Figure 7 once enclosed in the film of anti-adhesive material,
Figure 9 is a photograph taken with a Hitachi S-800 FEG scanning electron microscope, magnification x20, showing an embodiment of the second part of porous structure of an implant according to the invention, covered on one surface by the first film of anti-adhesive material,
Figures 10, 10A, 11 and 12 show embodiments of the knitting structure suitable for producing a textile for an implant according to the invention,
Figure 13 is a cross-sectional view of an implant according to the invention once it has been implanted after a direct colostomy,
Figure 14 is a schematic plan view of another embodiment of an implant according to the invention once it has been implanted after an indirect colostomy,
Figure 15 is a cross-sectional view of the implant from Figure 14 along the line II in Figure 14.

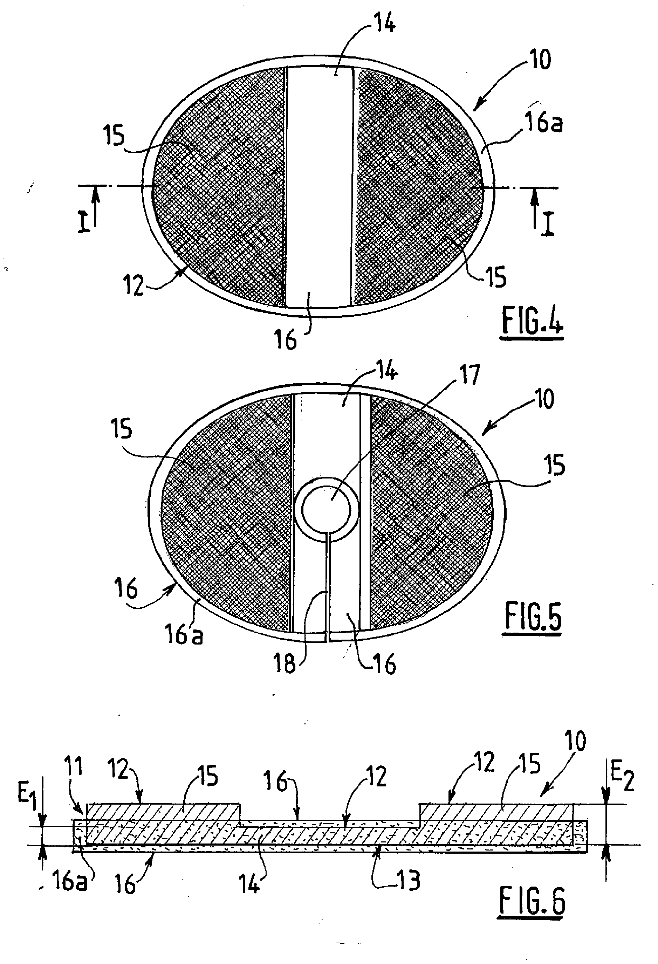

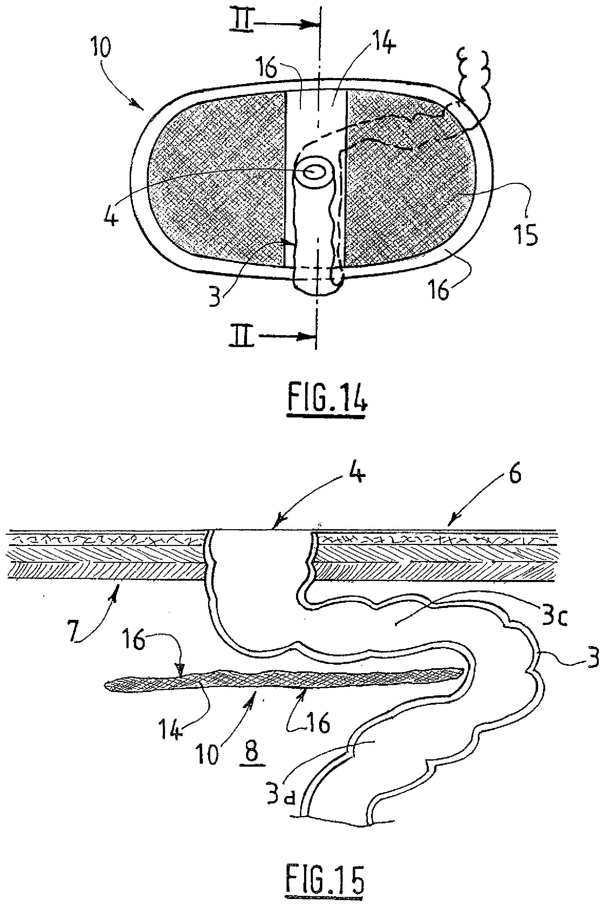

Referring to Figures 4 and 6, an implant 10 according to the invention is shown which comprises a layer of porous structure in the form of a biocompatible textile 11. As will be seen more clearly from Figures 13 and 15, the layer of porous structure or textile 11 comprises a first surface 12 intended to be placed facing the abdominal wall after implantation, and a second surface opposite the first surface 12, this second surface 13 being intended to be placed facing the abdominal cavity after implantation.

As will be seen clearly from Figure 4, which is a plan view of an implant according to the invention, the layer of porous structure comprises a first textile part 14 and a second textile part 15, the first textile part and the second textile part together forming the biocompatible textile 11 (see Figure 6). As will be seen more clearly from Figures 13-15 regarding the first surface 12 of the biocompatible textile 11, the first part 14 of textile is able to come into contact with the intestine, and the second part 15 of textile is intended to be placed facing the abdominal wall once the implant 10 according to the invention is implanted in the patient.

The implant 10 shown in Figure 4 is oval in shape. Its length can range, for example, from 15 to 30 cm, and its width can range, for example, from 12 to 20 cm. The shape of the implant can be adapted to the anatomy of the patient. It can also vary depending on the surgical technique envisaged.

In one example not shown, the implant has a generally round shape. Its diameter can then range from 5 to 20 cm, for example.

Referring to Figure 6, the implant 10 according to the invention is covered on its second surface 13 by a film 16 of anti-adhesive material. The edge 16a of the film of anti-adhesive material extends past the second surface 13 of the textile 11, for example by a distance ranging from 3 to 10 mm. Thus, the implant 10 is protected from contact with the viscera when it is implanted.

Figure 6 is a simplified cross-sectional view of the implant from Figure 4 along line II, where the thickness of the film 16 is exaggerated to make matters easier to understand. As will be seen clearly from Figure 6, the first part 14 of textile and the second part 15 of textile each have a thickness, namely a thickness E1 and a thickness E2, respectively. The value of the thickness E2 of the second part 15 of textile is superior to the value of the thickness E1 of the first part 14 of textile. Moreover, the film 16 completely encompasses the first part 14 of textile but only penetrates superficially into the thickness E2 of the second part 15 of textile. In Figure 6, the thickness of the film 16 is exaggerated. It must be understood that the film 16 penetrates into the second part 15 of textile only by a short distance, for example by a distance corresponding to 2 to 10% of the thickness E2. In the example shown, the value of the thickness E1 is 0.75 mm, while that of the thickness E2 is 2.00 mm.

Thus, as will be seen clearly from Figure 6, the first part 14 of textile is covered by film 16 of anti-adhesive material on its two surfaces, and this first part 14 of textile is totally enclosed within the film 16 of anti-adhesive material.

By contrast, as regards the second part 15 of textile, its first surface 12, intended to be placed facing the abdominal wall, is not covered by film 16 of anti-adhesive material. This surface 12 will be referred to hereinbelow as the open surface of the second part 15 of textile. By contrast, the second surface 13 intended to be placed facing the abdominal cavity, is covered by film 16 of anti-adhesive material. This surface 13 will be referred to hereinbelow as the closed surface of the second part of textile. Thus, the film 16 of anti-adhesive material penetrates only superficially into the second part 15 of textile, in the area of its closed surface 13, leaving open the porosity of the first open surface 12 of the second textile part 15.

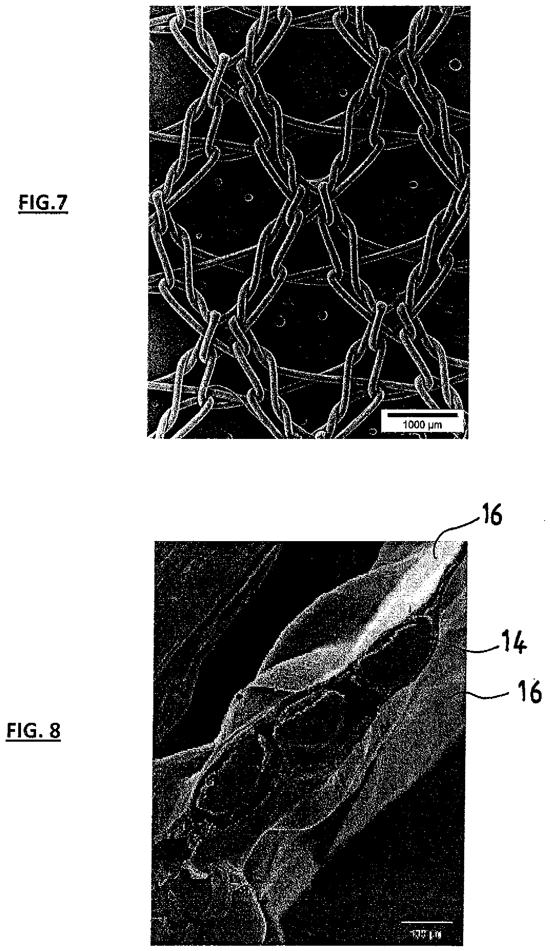

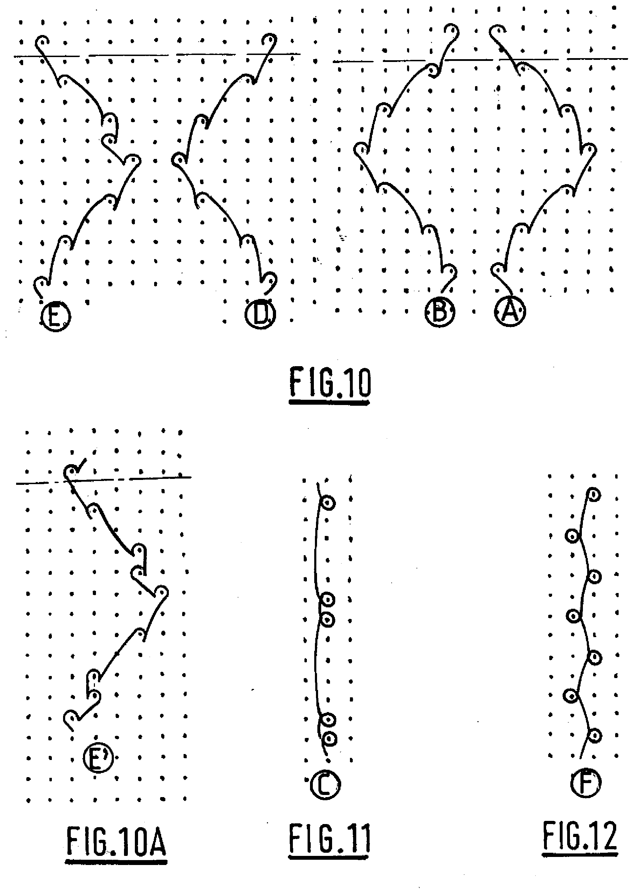

Figure 7 shows a view of the first part 14 of textile. In this example, the first part of textile is a knit obtained on a warp knitting machine or raschel machine with two guide bars A and B, threaded regularly with one guide full, one guide empty, using the knitting structure shown in Figure 10 for bars A and B. The respective charts used for bars A and B are the following:
Bar A: 4-4-5-4/4-4-4-3/3-3-2-1/1-1-0-1/1-1-1-2/2-2-3-4//
Bar B: 1-1-0-1/1-1-1-2/2-2-3-4/4-4-5-4/4-4-4-3/3-3-2-1//

The yarn used is preferably a monofilament yarn of polyethylene terephthalate, diameter 0.08 mm and titre 69 dtex. The knit thus formed comprises two opposite surfaces but is free of connecting sheets between its two opposite surfaces. It is a two-dimensional knit according to the present application.

The thickness of the first part of textile formed from such a knit is approximately 0.25 mm.

In the example shown, the knitting used for the first part of textile creates pores, preferably with dimensions that can range from 0.1 to 3 mm, preferably from 1.5 to 2 mm. At the moment of implantation, these pores are not visible, nor are they accessible to tissue colonization, because the whole of the first part of textile is confined in the film 16 of anti-adhesive material. However, after a few days, as the film of anti-adhesive material is absorbed and disappears after performing its function of limiting and/or avoiding formation of adhesions during the first 10 days following the implantation operation, the pores of the first part 14 of textile become accessible to tissue colonization. When a yarn of polyethylene terephthalate is used for producing the two-dimensional knit, this knit is non-bioabsorbable and remains permanently at the implantation site.

In another embodiment of the invention, said first part 14 of textile is made of a bioabsorbable material that is absorbed more slowly than the bioabsorbable material constituting the film 16 of anti-adhesive material.

As is shown in Figure 8, which is a scanning electron microscope photograph of a section of the implant according to one embodiment of the invention in the area of the first textile part, the latter is enclosed in the film 16 of anti-adhesive material. The coating of the first part 14 of textile by the film 16 of anti-adhesive material can be effected using any method known to a person skilled in the art. In the example shown in Figure 8, the first part 14 of textile is coated using the method described in the application WO2004/043294.

Thus, as will be seen clearly from Figure 8, the first part 14 of textile is covered by the film of anti-adhesive material on its two surfaces, and the porosity (see Figure 7) of the first part of textile is totally occluded at the moment of implantation. Thus, once covered with a film 16 of anti-adhesive material, the two surfaces of the first part 14 of textile are smooth and non-porous, as shown in Figure 8. The two surfaces of the first part 14 of textile do not damage the organs situated in the proximity of this first part 14 of textile, particularly the stoma organs.

The second part 15 of textile, of which the thickness is greater than that of the first part 14 of textile, can be a knit which is obtained on a warp knitting machine or double-bed raschel machine and which has two opposite surfaces connected to each other by connecting yarns, that is to say a three-dimensional knit according to the present application. For example, a first surface of the knit is produced with the two guide bars A and B already mentioned above for producing the first part 14 of textile, these being threaded identically and with the same charts as above. The second surface of the knit is produced with two supplementary guide bars D and E, threaded with one guide full, one guide empty, using the knitting structure shown in Figure 10 for bars D and E. The respective charts used for bars D and E are the following:
Bar D: 0-1-1-1/1-2-2-2/3-4-4-4/5-4-4-4/4-3-3-3/2-1-1-1//
Bar E: 5-4-4-4/4-3-3-3/2-1-1-1/0-1-1-1/1-2-2-2/3-4-4-4//

The connection of the two surfaces can be effected, for example, by hooking one loop in two, or in three, or in four, or in five, or in six of one of the bars D or E, whose knitting structure will be adapted. For example, in one embodiment of the invention, the connection of the two surfaces is effected by hooking one loop in three of the bar E, which thus becomes bar E', with the knitting structure shown in Figure 10A and according to the following chart:
Bar E': 5-4-3-4/4-3-3-3/2-1-1-1/0-1-2-1/1-2-2-2/3-4-4-4//

In another embodiment, the connection of the two surfaces can be effected with the aid of a fifth guide bar C, with the knitting structure shown in Figure 11 and according to the following chart:
Bar C: 1-0-1-0/1-1-1-1/1-1-1-1//

Thus, when the first part 14 of textile is in the form of a central strip separating two lateral strips of the second part 15 of textile, as is shown in Figures 4 and 5, the textile 11 can be produced in one piece, on the same knitting machine. With the guide bars A, B, D and E' described above:
- the whole of the first surface 13 of the textile 11 is produced with the two guide bars A and B,
- along a first length, corresponding to the first lateral strip of the second part 15 of textile, the guide bars D and E' are threaded one guide full, one guide empty, in order to produce the second surface of the three-dimensional knit forming said second part 15 of textile,
- then, along the length corresponding to the width of the central strip of the first part 14 of textile, the guide bars D and E' are left empty in order to form the two-dimensional knit,
- finally, along a length corresponding to the second lateral strip of the second part 15 of textile, the guide bars D and E' are again threaded one guide full, one guide empty, in order to produce the second surface of the three-dimensional knit forming said second part 15 of textile.

In such a case, the optional fifth guide bar C is threaded only in the zones of the three-dimensional knit.

Finally, in order to obtain a smooth join between the three-dimensional knit forming the second part 15 of textile and the two-dimensional knit forming the first part 14 of textile, it is possible to use, still on the same knitting machine, a supplementary guide bar F in order to finish the edges of the three-dimensional knits, threaded in the area of these edges, according to the knitting structure shown in Figure 12, using the following chart for example:
Bar F: 1-0-1-1/1-2-1-1//

A monofilament yarn will preferably be chosen to produce the second part 15 of textile. This is because multifilament yarns pose greater risks of bacteria developing in the interstices present between the various filaments of the yarn.

The yarn used is preferably a monofilament yarn of polyethylene terephthalate, with a diameter of approximately 0.08 mm and titre 69 dtex.

The thickness of the second part 15 of textile, produced in the form of the three-dimensional knit described above, is approximately 1.50 mm.

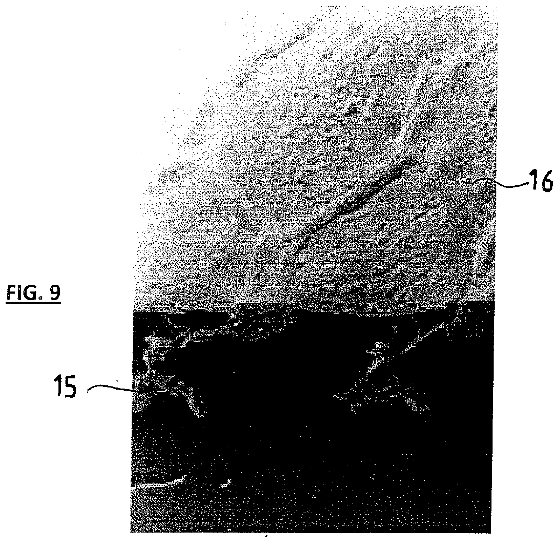

As will be seen from Figure 9, the second part 15 of textile is covered, on its surface intended to face the abdominal cavity, by the film 16 of anti-adhesive material. The film 16 of anti-adhesive material penetrates only superficially into the three-dimensional knit forming the second part 15 of textile. Consequently, the surface of the second part 15 of textile intended to face the abdominal wall is open, and its porosity is not occluded. This open surface therefore promotes all cellular growth.

The superficial covering of the surface of the second part 15 of textile intended to be placed facing the abdominal cavity can be carried out using any method known to a person skilled in the art, for example using the method described in the application WO99/06080.

The material used for the film 16 of anti-adhesive material can, for example, be collagen prepared in the manner described in the application WO99/06080.

The film 16 of anti-adhesive material may be applied to the surface of the textile 11 intended to be placed facing the abdominal cavity, in the following way:
- The solution of collagen is poured into a mould having the external dimensions desired for the film. The textile produced above is then applied to this solution, at the centre of the mould, the surface to be covered being placed on the solution of collagen. The solution of collagen then penetrates into the textile by capillary force, completely coating the first part of textile and covering the latter on the two opposite surfaces of the two-dimensional knit forming it, and penetrating only by a small distance into the thickness of the second part of textile, thus creating a superficial film for this three-dimensional part. Once the collagen has dried, the film is cut around the textile using a scalpel.

Alternatively, the covering/coating method described in WO2004/043294 can be used.

In another embodiment not shown here, the film 16 only superficially covers the surface of the first part of textile, intended to be placed facing the abdominal cavity, and does not encompass the two opposite surfaces of this first part of textile. In such a case, the surface of the first part of textile intended to be placed facing the abdominal wall is covered with a second film of anti-adhesive material. Thus, each of the two opposite surfaces of the first part of textile is covered by a smooth and continuous film of anti-adhesive material. Covering methods that can be used to form this second film are also described in WO2004/043294.

Figure 13 shows an implant according to the invention after it has been implanted, in the case of a direct stoma. To do this, the implant according to the invention shown in Figure 5 is used for example. In this figure, the reference numbers designating the same elements as in Figure 4 have been retained. The implant 10 in Figure 5 comprises an orifice 17 which has been created at the centre of the implant 10 and at the centre of the central strip formed by the first part 14 of textile. Such an orifice 17 can have a diameter ranging from 1 to 8 cm. A slit 18 starting from the central orifice 17 and opening out on an edge of the implant 10 allows the implant to be adjusted around the colon 3 during implantation of the implant.

In one embodiment not shown here, the orifice 17 is offset relative to the centre of the implant 10. It is also possible to have several orifices, depending on the surgery envisaged.

Thus, in Figure 13, an implant 10 similar to that in Figure 5 has been placed around the colon 3, which is at right angles to the abdominal wall 7 and to the skin 6. As will be seen from this figure, the first part 14 of textile covered entirely, that is to say on its two opposite surfaces, by the film 16 of anti-adhesive material is situated in direct proximity to the colon 3. Thus, the colon 3, which is a fragile organ, is not damaged by the implant 10. The open surface of the second part 15 of textile, which is porous and promotes cellular recolonization, is situated facing the abdominal wall 7. Thus, after implantation, the cells of the abdominal wall can gradually colonize the second part 15 of textile, for example the three-dimensional knit forming it.

It is possible to fix the implant 10 to the abdominal wall 7 using staples or sutures. In addition, or alternatively, the open surface of the second part 15 of textile can intrinsically comprise barbs or loops, which will facilitate its natural attachment to the abdominal wall. Such an affixing knit is described in the application WO01/81667.

Finally, the second surface of the textile, completely covered by film 16 of anti-adhesive material, is situated facing the abdominal cavity 8. Thus, the hollow and fragile organs, the viscera, are not damaged by the implant.

Figures 14 and 15 show an implant according to the invention after it has been implanted, in the case of an indirect stoma. To do this, the implant according to the invention in Figure 4 is used, for example. Figure 14 shows a plan view of the implant 10 according to Figure 4 at its implantation site in the area of the colon 3. For greater clarity, the skin and the abdominal wall have not been depicted. As will be seen from Figure 15, which is a cross-sectional view of Figure 14 along line II-II and in which the abdominal wall 7 and the skin 6 have been depicted, the colon 3 forms a bend prior to exteriorization, and the implant 10 is placed inside this bend. A part 3c of the colon is thus situated between the implant 10 and the abdominal wall 7.

As will be seen from these two figures, the part 3c of the colon faces and is able to come into contact with the first part 14 of textile covered on its two opposite surfaces by the film 16 of anti-adhesive material. Thus, neither the part 3c of the colon, situated between the implant 10 and the abdominal wall 7, nor the part 3d of the colon corresponding to the second length of the bend and able to lie under the implant 10 in the area of the abdominal cavity 8, risks being damaged by the implant 10. This is because the parts 3c and 3d of the colon 3 are each facing a surface of the first part 14 of textile covered by a film 16 of anti-adhesive material. Moreover, the relatively small thickness E1 of this first part 14 of textile permits flexible and atraumatic support of the colon 3.

In an indirect stoma of this kind, the implant 10 essentially acts like a hammock for the part 3c of the colon 3, and the implant 10 can be fixed to the abdominal wall 7 via the open surface of the second part 15 of textile placed facing the abdominal wall 7.

The present disclosure also relates to a method for treatment or prevention of a hernia in the proximity of a stoma formed in the skin, comprising the step of implanting an implant of the type described above in the area of the stoma. In one embodiment of the invention, the implant is fixed to the abdominal wall.

The implant described above can be implanted by open surgery or by laparoscopy.

The implant according to the invention is used in particular in the treatment of parastomal hernias. It is able to support and/or protect the organs that are to be treated, such as the colon or ureters, without damaging them, while at the same time effectively strengthening the wall in which the stoma is formed, such as the abdominal wall, irrespective of the type of stoma formed, i.e. direct stoma or indirect stoma.

## Claims

1. An implant (10) for the prevention of a hernia formed in the abdominal wall (7) in the proximity of a stoma (4) of an organ (3), comprising: a biocompatible textile (11) including a first surface (12) intended to face an abdominal wall and a second surface (13) intended to face an abdominal cavity, the second surface opposite the first surface, the biocompatible textile including a first textile part (14) intended to be in contact with the stoma of an organ and including a two-dimensional knit and having a first thickness E1, a second textile part (15) including a three-dimensional knit and having a second thickness E2 greater than first thickness E1, a first anti-adhesive film (16) covering the second surface (13) of the biocompatible textile wherein the first textile part (14) is also covered by a second anti-adhesive film (16) on the first surface (12).

2. The implant (10) according to claim 1, wherein the first film and the second film form a single and unique film (16).

3. The implant (10) according to any one of claims 1 to 2, wherein the first surface of the biocompatible textile along the second textile part has an open surface.

4. The implant (10) according to any one of claims 1-3, wherein the first thickness E1 is from approximately 0.15 to approximately 0.50 mm.

5. The implant (10) according to any one of claims 1-4, wherein the second thickness E2 is from about 0.40 to about 3.00 mm.

6. The implant (10) according to any one of claims 1 to 5, wherein the biocompatible textile includes pores with dimensions of from approximately 0.1 to approximately 3 mm.

7. The implant (10) according to any one of claims 1 to 6, wherein the first and second textile parts are composed of at least one yarn made of biocompatible materials, bioabsorbable materials, non-bioabsorbable materials and their mixtures.

8. The implant (10) according to claim 7, wherein the bioabsorbable materials are chosen from among polylactic acid (PLA), polysaccharides, polycaprolactones (PCL), polydioxanones (PDO), trimethylene carbonates (TMC), polyvinyl alcohol (PVA), polyhydroxyalkanoates (PHA), polyamides, polyethers, oxidized cellulose, polyglycolic acid (PGA), copolymers of these materials and their mixtures.

9. The implant (10) according to claim 7, wherein the non-bioabsorbable materials are chosen from among polypropylenes, polyesters such as polyethylene terephthalates, polyamides, polyvinylidene fluoride, and their mixtures.

10. The implant (10) according to any one of claims 7-9, wherein the at least one yarn is chosen from among monofilament yarns, multifilament yarns and their combinations.

11. The implant (10) according to any one of claims 7-10, wherein the at least one yarn forming the first textile part is a monofilament yarn including polyethylene terephthalate.

12. The implant (10) according to any one of claims 7-11, wherein the at least one yarn forming the second textile part is a monofilament yarn including polyethylene terephthalate.

13. The implant (10) according to any one of claims 1-12, wherein the first textile part (14) represents a central strip of the biocompatible textile and the second textile part (15) represents lateral strips of the biocompatible textile.

14. The implant (10) according to any one of claims 1-13, wherein the two-dimensional knit of the first textile part and the three-dimensional knit of the second textile part are joined together by at least one seam.

15. The implant (10) according to any one of claims 1-13, wherein the two-dimensional knit of the first textile part and the three-dimensional knit of the second textile part are knitted together on the same knitting machine and constitute a textile made in one piece.

16. The implant (10) according to any one of claims 1-15, wherein the first surface of the biocompatible textile along the second textile part further includes elements chosen from loops, barbs, hooks, threads, or clips for fastening the second textile part to the abdominal wall.

17. The implant (10) according to any one of claims 1-16, wherein the first and/or second anti-adhesive films comprise a material chosen from among bioabsorbable materials, non-bioabsorbable materials and their mixtures.

18. The implant (10) according to claim 17, wherein the bioabsorbable materials are chosen from among collagens, oxidized celluloses, polyarylates, trimethylene carbonates, caprolactones, dioxanones, glycolic acid, lactic acid, glycolides, lactides, polysaccharides, chitosans, polyglucuronic acids, hylauronic acids, dextrans and their mixtures.

19. The implant (10) according to claim 17, wherein the non-bioabsorbable materials are chosen from among polytetrafluoroethylene, polyethylene glycols, polysiloxanes, polyurethanes, stainless steels, derivatives of precious metals and their mixtures.

20. The implant (10) according to claim 18, wherein the bioabsorbable material is a hydrophilic material chosen from the group consisting of collagens, polysaccharides and their mixtures.

21. The implant (10) according to any one of claims 1-20, wherein the first anti-adhesive film extends past an edge of the biocompatible textile (11).

22. The implant (10) according to claim 21, wherein the first anti-adhesive film extends past the edge of the biocompatible textile by about 3 to about 10 millimeters.

23. The implant (10) according to claim 13, further comprising at least one orifice (17) formed at about a center of the first textile part to provide a passage for a stoma organ during implantation of the implant.

24. The implant (10) according to claim 13, further comprising at least one orifice formed within the first textile part, the orifice being offset relative to the center of the implant.

## Patentansprüche

1. Implantat (10) für die Vorbeugung einer Hernie, die in der Bauchdecke (7) in der Nähe eines Stomas (4) eines Organs (3) ausgebildet ist, das Folgendes umfasst:
eine biokompatible Textilie (11), die eine erste Oberfläche (12), die dazu bestimmt ist, einer Bauchdecke zugewandt zu sein, und eine zweite Oberfläche (13) beinhaltet, die dazu bestimmt ist, einer Bauchhöhle zugewandt zu sein, wobei die zweite Oberfläche der ersten Oberfläche gegenüberliegt, wobei die biokompatible Textilie einen ersten Textilienteil (14), der dazu bestimmt ist, mit dem Stoma eines Organs in Berührung zu stehen, und der eine zweidimensionalen Masche beinhaltet und eine erste Dicke E1 aufweist, einen zweiten Textilienteil (15), der eine dreidimensionale Masche beinhaltet und eine zweite Dicke E2 aufweist, die größer als die erste Dicke E1 ist, eine erste Antihaftfolie (16) beinhaltet, die die zweite Oberfläche (13) der biokompatiblen Textilie bedeckt, wobei der erste Textilienteil (14) ebenso von einer zweiten Antihaftfolie (16) auf der ersten Oberfläche (12) bedeckt ist.

2. Implantat (10) nach Anspruch 1, wobei die erste Folie und die zweite Folie eine einzelne und einzigartige Folie (16) ausbilden.

3. Implantat (10) nach einem der Ansprüche 1 bis 2, wobei die erste Oberfläche der biokompatiblen Textilie entlang des zweiten Textilienteils eine offene Oberfläche aufweist.

4. Implantat (10) nach einem der Ansprüche 1-3, wobei die erste Dicke E1 von ungefähr 0,15 bis ungefähr 0,50 mm beträgt.

5. Implantat (10) nach einem der Ansprüche 1-4, wobei die zweite Dicke E2 von etwa 0,40 bis etwa 3,00 mm beträgt.

6. Implantat (10) nach einem der Ansprüche 1 bis 5, wobei die biokompatible Textilie Poren mit Abmessungen von ungefähr 0,1 bis ungefähr 3 mm beinhaltet.

7. Implantat (10) nach einem der Ansprüche 1 bis 6, wobei der erste und der zweite Textilienteil aus wenigstens einem Faden aus biokompatiblen Materialien, bioabsorbierbaren Materialien, nicht bioabsorbierbaren Materialien und deren Mischungen bestehen.

8. Implantat (10) nach Anspruch 7, wobei die bioabsorbierbaren Materialien aus Polymilchsäure (*polylactic acid* - PLA), Polysacchariden, Polycaprolactonen (PCL), Polydioxanonen (PDO), Trimethylencarbonaten (TMC), Polyvinylalkohol (PVA), Polyhydroxyalkanoaten (PHA), Polyamiden, Polyethern, oxidierter Cellulose, Polyglycolsäure (*polyglycolic acid* - PGA), Copolymeren dieser Materialien und deren Mischungen ausgewählt sind.

9. Implantat (10) nach Anspruch 7, wobei die nicht bioabsorbierbaren Materialien aus Polypropylenen, Polyestern, wie etwa Polyethylenterephthalaten, Polyamiden, Polyvinylidenfluorid und deren Mischungen ausgewählt sind.

10. Implantat (10) nach einem der Ansprüche 7-9, wobei der wenigstens eine Faden aus Monofilamentfäden, Multifilamentfäden und deren Kombinationen ausgewählt ist.

11. Implantat (10) nach einem der Ansprüche 7-10, wobei der wenigstens eine Faden, der den ersten Textilienteil ausbildet, ein Monofilamentfaden ist, der Polyethylenterephthalat beinhaltet.

12. Implantat (10) nach einem der Ansprüche 7-11, wobei der wenigstens eine Faden, der den zweiten Textilienteil ausbildet, ein Monofilamentfaden ist, der Polyethylenterephthalat beinhaltet.

13. Implantat (10) nach einem der Ansprüche 1-12, wobei der erste Textilienteil (14) einen mittleren Streifen der biokompatiblen Textilie darstellt und der zweite Textilienteil (15) seitliche Streifen der biokompatiblen Textilie darstellt.

14. Implantat (10) nach einem der Ansprüche 1-13, wobei die zweidimensionale Masche des ersten Textilienteils und die dreidimensionale Masche des zweiten Textilienteils durch wenigstens eine Naht miteinander verbunden sind.

15. Implantat (10) nach einem der Ansprüche 1-13, wobei die zweidimensionale Masche des ersten Textilienteils und die dreidimensionale Masche des zweiten Textilienteils auf derselben Strickmaschine zusammengestrickt sind und eine in einem Stück hergestellte Textilie ausmachen.

16. Implantat (10) nach einem der Ansprüche 1-15, wobei die erste Oberfläche der biokompatiblen Textilie entlang des zweiten Textilienteils ferner Elemente beinhaltet, die aus Schlaufen, Widerhaken, Haken, Garnen oder Klammern zum Befestigen des zweiten Textilienteils an der Bauchdecke ausgewählt sind.

17. Implantat (10) nach einem der Ansprüche 1-16, wobei die erste und/oder die zweite Antihaftfolie ein Material umfassen, das aus bioabsorbierbaren Materialien, nicht bioabsorbierbaren Materialien und deren Mischungen ausgewählt ist.

18. Implantat (10) nach Anspruch 17, wobei die bioabsorbierbaren Materialien aus Kollagenen, oxidierten Cellulosen, Polyarylaten, Trimethylencarbonaten, Caprolactonen, Dioxanonen, Glycolsäure, Milchsäure, Glycoliden, Lactiden, Polysacchariden, Chitosanen, Polyglucuronsäuren, Hylauronsäuren, Dextranen und deren Mischungen ausgewählt sind.

19. Implantat (10) nach Anspruch 17, wobei die nicht bioabsorbierbaren Materialien aus Polytetrafluorethylen, Polyethylenglycolen, Polysiloxanen, Polyurethanen, rostfreien Stählen, Derivaten von Edelmetallen und deren Mischungen ausgewählt sind.

20. Implantat (10) nach Anspruch 18, wobei das bioabsorbierbare Material ein hydrophiles Material ist, das aus der Gruppe ausgewählt ist, die aus Kollagenen, Polysacchariden und deren Mischungen besteht.

21. Implantat (10) nach einem der Ansprüche 1-20, wobei sich die erste Antihaftfolie über einen Rand der biokompatiblen Textilie (11) hinaus erstreckt.

22. Implantat (10) nach Anspruch 21, wobei sich die erste Antihaftfolie um etwa 3 bis etwa 10 Millimeter über den Rand der biokompatiblen Textilie hinaus erstreckt.

23. Implantat (10) nach Anspruch 13, das ferner wenigstens eine Öffnung (17) umfasst, die etwa in der Mitte des ersten Textilienteils ausgebildet ist, um während einer Implantation des Implantats einen Durchgang für ein Stomaorgan bereitzustellen.

24. Implantat (10) nach Anspruch 13, das ferner wenigstens eine Öffnung umfasst, die innerhalb des ersten Textilienteils ausgebildet ist, wobei die Öffnung relativ zu der Mitte des Implantats versetzt ist.

## Revendications

1. Implant (10) destiné à la prévention d'une hernie formée dans la paroi abdominale (7) à proximité d'une stomie (4) d'un organe (3), comprenant :
un textile biocompatible (11) comportant une première surface (12) destinée à faire face à une paroi abdominale et une seconde surface (13) destinée à faire face à une cavité abdominale, la seconde surface étant opposée à la première surface, le textile biocompatible comportant une première partie textile (14) destinée à être en contact avec la stomie d'un organe et comportant un tricot bidimensionnel et présentant une première épaisseur E1, une second partie textile (15) comportant un tricot tridimensionnel et présentant une seconde épaisseur E2 supérieure à la première épaisseur E1, un premier film antiadhésif (16) couvrant la seconde surface (13) du textile biocompatible, la première partie textile (14) étant également couverte d'un second film antiadhésif (16) sur la première surface (12).

2. Implant (10) selon la revendication 1, dans lequel le premier film et le second film forment un seul film unique (16).

3. Implant (10) selon l'une quelconque des revendications 1 à 2, dans lequel la première surface du textile biocompatible le long de la seconde partie textile présente une surface ouverte.

4. Implant (10) selon l'une quelconque des revendications 1 à 3, dans lequel la première épaisseur E1 est d'environ 0,15 à environ 0,50 mm.

5. Implant (10) selon l'une quelconque des revendications 1 à 4, dans lequel la seconde épaisseur E2 est d'environ 0,40 à environ 3,00 mm.

6. Implant (10) selon l'une quelconque des revendications 1 à 5, dans lequel le textile biocompatible comprend des pores d'environ 0,1 à environ 3 mm.

7. Implant (10) selon l'une quelconque des revendications 1 à 6, dans lequel les première et seconde parties textiles sont composées d'au moins un fil fabriqué à partir de matériaux biocompatibles, de matériaux bioabsorbables, de matériaux non bioabsorbables et de leurs mélanges.

8. Implant (10) selon la revendication 7, dans lequel les matériaux bioabsorbables sont choisis parmi l'acide polylactique (PLA), les polysaccharides, les polycaprolactones (PCL), les polydioxanones (PDO), les carbonates de triméthylène (TMC), l'alcool polyvinylique (PVA), les polyhydroxyalcanoates (PHA), les polyamides, les polyéthers, la cellulose oxygénée, l'acide polyglycolique (PGA), les copolymères de ces matériaux et leurs mélanges.

9. Implant (10) selon la revendication 7, dans lequel les matériaux non bioabsorbables sont choisis parmi les polypropylènes, les polyesters tels que les téréphtalates de polyéthylène, les polyamides, le fluorure de polyvinylidène et leurs mélanges.

10. Implant (10) selon l'une quelconque des revendications 7 à 9, dans lequel au moins un fil est choisi parmi les fils monofilaments, les fils multifilaments et leurs combinaisons.

11. Implant (10) selon l'une quelconque des revendications 7 à 10, dans lequel l'au moins un fil formant la première partie textile est un fil monofilament comportant le téréphtalate de polyéthylène.

12. Implant (10) selon l'une quelconque des revendications 7 à 11, dans lequel l'au moins un fil formant la seconde partie textile est un fil monofilament comportant le téréphtalate de polyéthylène.

13. Implant (10) selon l'une quelconque des revendications 1 à 12, dans lequel la première partie textile (14) représente une bande centrale du textile biocompatible et la seconde partie textile (15) représente des bandes latérales du textile biocompatible.

14. Implant (10) selon l'une quelconque des revendications 1 à 13, dans lequel le tricot bidimensionnel de la première partie textile et le tricot tridimensionnel de la seconde partie textile sont reliés ensemble par au moins une couture.

15. Implant (10) selon l'une quelconque des revendications 1 à 13, dans lequel le tricot bidimensionnel de la première partie textile et le tricot tridimensionnel de la seconde partie textile sont tricotés ensemble sur la même machine à tricoter et constituent un textile fabriqué en une seule pièce.

16. Implant (10) selon l'une quelconque des revendications 1 à 15, dans lequel la première surface du textile biocompatible le long de la seconde partie textile comporte également des éléments choisis parmi des boucles, des ardillons, des crochets, des fils ou des pinces destinés à attacher la seconde partie textile sur la paroi abdominale.

17. Implant (10) selon l'une quelconque des revendications 1 à 16, dans lequel les premier et/ou second films antiadhésifs comprennent un matériau choisi parmi les matériaux bioabsorbables, les matériaux non bioabsorbables et leurs mélanges.

18. Implant (10) selon la revendication 17, dans lequel les matériaux bioabsorbables sont choisis parmi les collagènes, les celluloses oxydées, les polyarylates, les carbonates de triméthylène, les caprolactones, les dioxanones, l'acide glycolique, l'acide lactique, les glycolides, les lactides, les polysaccharides, les chitosanes, les acides polyglucuroniques, les acides hyaluroniques, les dextranes et leurs mélanges.

19. Implant (10) selon la revendication 17, dans lequel les matériaux non bioabsorbables sont choisis parmi le polytétrafluoroéthylène, les polyéthylènes glycols, les polysiloxanes, les polyuréthanes, les aciers inoxydables, les dérivés des métaux précieux et leurs mélanges.

20. Implant (10) selon la revendication 18, dans lequel le matériau bioabsorbable est un matériau hydrophile choisi dans le groupe composé de collagènes, de polysaccharides et de leurs mélanges.

21. Implant (10) selon l'une quelconque des revendications 1 à 20, dans lequel le premier film antiadhésif s'étend au-delà du bord du textile biocompatible (11).

22. Implant (10) selon la revendication 21, dans lequel le premier film antiadhésif s'étend au-delà du bord du textile biocompatible d'environ 3 à environ 10 millimètres.

23. Implant (10) selon la revendication 13, comprenant en outre au moins un orifice (17) formé à environ un centre de la première partie textile pour fournir un passage pour un organe stomisé pendant l'implantation de l'implant.

24. Implant (10) selon la revendication 13, comprenant en outre au moins un orifice formé à l'intérieur de la première partie textile, l'orifice étant décalé par rapport au centre de l'implant.
